# EUROPEAN PATENT SPECIFICATION

(11) **EP 1 224 221 B1**
(45) Date of publication and mention of the grant of the patent: **15.12.2004**
(21) Application number: 00973314.8
(22) Date of filing: 19.10.2000
(51) Int. Cl.: C07K 14/705

(54) **TRANSCRIPTION FACTOR-SELECTIVE NUCLEAR TRANSPORT RECEPTORS REQUIRED FOR IMMUNE RESPONSE ACTIVATION**
FüR DIE AKTIVIERUNG DER IMMUNANTWORT BENOETIGTE TRANSKRIPTIONSFAKTOR-SELEKTIVE, NUKLEAERE TRANSPORTREZEPTOREN
RECEPTEURS NUCLEAIRES SELECTIFS DE TRANSPORT DE FACTEURS DE TRANSCRIPTION NECESSAIRES POUR ACTIVER LA REPONSE IMMUNITAIRE

(30) Priority: 22.10.1999 SE 9903832
(43) Date of publication of application: 24.07.2002
(73) Proprietor: Innate Pharmaceuticals AB, 903 47 Umea (SE)
(72) Inventor: SAMAKOVLIS, Christos, S-907 38 Umea (SE); UV, Anne, Elisabeth, S-907 51 Umea (SE)
(74) Representative: Johansson, Lars E.
(86) International application number: PCT/SE2000/002022
(87) International publication number: WO 2001/029087

(56) References cited:
- MAARTEN FORNEROD ET AL.: 'The human homologue of yeast CRM1 is in a dynamic subcomplex with CAN/Nup214 and a novel nuclear pore component Nup88' THE EMBO JOURNAL vol. 16, no. 4, 1997, pages 807 - 816, XP002936124 & EMBL/Genbank/DDBJ, AC= Y08612
- RICARDO BASTOS ET AL.: 'Nup84, a novel nucleoporin that is associated with CAN/Nup214 on the cytoplasmic face of the nuclear pore complex' THE JOURNAL OF CELL BIOLOGY vol. 137, no. 5, June 1997, pages 989 - 1000, XP002936125 & EMBL/Genbank/DDBJ, AC= U93692
- JUDITH BOER ET AL.: 'Overexpression of the nucleoporin CAN/Nup214 induces growth arrest, nucleocytoplasmic transport defects and apoptosis' MOLECULAR AND CELLULAR BIOLOGY vol. 18, no. 3, March 1998, pages 1236 - 1247, XP002936126
- EMBL/Genbank/DDBJ databases, accession no. AY004880, UV A.E. et al: "Members only encodes a drosophila nucleoporin required for rel protein import and immune response activation", 17 August 2000, XP002936127

### SEQUENCE LISTING

<110> INNATE PHARMACEUTICALS AB
<120> TRANSCRIPTION FACTOR-SELECTIVE NUCLEAR TRANSPORT RECEPTORS REQUIRED FOR IMMUNE RESPONSE ACTIVATION
<130> UMG-0002.PCT
<160> 5
<170> FastSEQ for Windows Version 3.0
<210> 1
   <211> 2514
   <212> DNA
   <213> Drosophila
<400> 1
   tgacttacaa gccgggcaac gaattgaagc ataaacaaac gatgtcgctc accgatgtct
   60
   tggaattgaa caaaacggag ttgttcgcga agattcgcaa tgggttgccc gtggtgcaaa
   120
   ggactcagaa cctgctggac tgcaaggacg atctgctctt tgcctggcac gcgaaggaca
   180
   gctgtctgtt ggttcgcaac tggcgctcat cgctggcggc aaaggtgaat atccagttcc
   240
   agacactgat tccatcgagc ttggtgagcc tggagatgga ccgcgtgctg gcctccaacg
   300
   agggctccct cgtggcacta agtggaccgc gcggcgttgt cataatggag ctgccccgcc
   360
   gctggggccc cgatggatac tacaaggatg gcaagccagt gatcacctgc cgcacgttcg
   420
   gcctggacac tcagcttttc ctaaaaaacc cgcacttgga agtgcgccag gtccgctggc
   480
   atccgcactc cgtgtcagac tccacgctgc tcgtgctgct caacaacaac acgattcgag
   540
   tgtacaatca ctccaagttg cgccacgtgt ggcaggtggg tcccccggtg cttcgaagtg
   600
   gggccaataa ctcgctatgc gactttggcg agctagccgt tgactttgac atcgctcctg
   660
   ctgcaaaacc acgtgtcact gaaccagaaa cagccggcaa taacgaaacc actttggata
   720
   agagcaacaa aacccttgtg gcggccaaat cgctgccgaa acaggagagg atagagtggc
   780
   ccatggttgt gctccgcgag aatggaaaca tctacatttt gatgaccggc gtggattcgg
   840
   agaatacgcg cctgcagggc cccgttacca taacgccaca ggcacatgac aattatggac
   900
   tggagtcctg tgccctaatg attatcccat cacttccgcc gaccattgtt atagctgaat
   960
   ccaatggaaa gcttcaccat gctctgctta tggaggcgga ggccactgag cattctttca
   1020
   atgaggttga cgactctgtg ttgattgaac ccgccgaata cgttgtacat gtactagaaa
   1080
   ctgtcgaact tgagctggga atatctgcac ccgcaactgg aaaagaagga ggcaactgtc
   1140
   ccatatactt aaagcgggat ttgatcaatg acgttcgcta ctttgcctac cacaacgcag
   1200
   gacttcatgc cgttacggtc agctttatcg cagagctgca acgttacttg gaaagcgagt 1260
   ccgatgaaga tcgtctagag ctggcggtct ctgcaagtgc agagtacatt ctgtgcacaa 1320
   aattcgattc cagtgagacg gtgaacgcag tctttggact tgccctatta caaattcctg 1380
   ccggtatagt tctactgctg ggcagcggtc aggtgattag ccttaagctg gtaattgatg 1440
   cccagctgtt ggttacacct aatgagaata agccagttga ctcggaagtg tctcagaagg 1500
   aaagtgggcc accgtttgtc gatacgatta aatcgctttt gcaacgtagt gttaaccagc 1560
   ccatcttggc ggataaactt tcctcgccat ccgcccagga gagcttcgaa ctgcttaacc 1620
   aggcaatcga ggtgcttcgt gagcagtacc ttaaacgaca tgaccttgtg cgcgccgcat 1680
   ttacacgcca catcaaccag atccagctga agaaggaaca acagctgcag gagatccaag 1740
   acctggagca ggaacgcgaa ctgatcagcg agcgggccca caagcttgcc gagcgcttcg 1800
   aggagattag ctacaaccag gagctgctcg tccgcaagtg caacgctctg atgcagaggg 1860
   ccaatgcctc actacccaat agtgtgattg cagaacggga gttttcgcag gaggtgattc 1920
   gacttaataa ggtcacccag agcctggccg ctggactgga gactgctaag aagacgttta 1980
   acaagcagcg ctaccacatt gcccagagtc aagaggacct caagaagaac gcttacgagc 2040
   tgccggagaa acagcaccgc actatcaccg agatacttac ccagctaacc ggggagatcg 2100
   atcgccagat caccgacgtc aagcgcatta ataaaatcgt tggcatctaa attcggattg 2160
   ggaagcacgc tcttaagatt tcaccacgtt ttcgtgatag taaagccgtt gcgtagaact 2220
   aaaacgtatt tttaataaac atgtcaatta agtcctattt tcgttagctg gcttctgttt 2280
   tttgtattcg atgacacttt aatgagcata cgtcagcgtt ttctgctaga cgatccttca 2340
   tgaagacaca aaaacatttg atacccatgg gtattgtgct acaaagtcta ctacagtaaa 2400
   atctattctt gacgtactgt cccattttcc ggattatagt tcaaaaagta aaataatagt 2460
   aataaaataa taataataat aaaataatga attaataaaa aaaaaaaaaa aaaa 2514
<210> 2
   <211> 2109
   <212> DNA
   <213> Drosophila
<400> 2
   atgtcgctca ccgatgtctt ggaattgaac aaaacggagt tgttcgcgaa gattcgcaat
   60
   gggttgcccg tggtgcaaag gactcagaac ctgctggact gcaaggacga tctgctcttt
   120
   gcctggcacg cgaaggacag ctgtctgttg gttcgcaact ggcgctcatc gctggcggca
   180
   aaggtgaata tccagttcca gacactgatt ccatcgagct tggtgagcct ggagatggac
   240
   cgcgtgctgg cctccaacga gggctccctc gtggcactaa gtggaccgcg cggcgttgtc
   300
   ataatggagc tgccccgccg ctggggcccc gatggatact acaaggatgg caagccagtg
   360
   atcacctgcc gcacgttcgg cctggacact cagcttttcc taaaaaaccc gcacttggaa
   420
   gtgcgccagg tccgctggca tccgcactcc gtgtcagact ccacgctgct cgtgctgctc
   480
   aacaacaaca cgattcgagt gtacaatcac tccaagttgc gccacgtgtg gcaggtgggt
   540
   cccccggtgc ttcgaagtgg ggccaataac tcgctatgcg actttggcga gctagccgtt
   600
   gactttgaca tcgctcctgc tgcaaaacca cgtgtcactg aaccagaaac agccggcaat
   660
   aacgaaacca ctttggataa gagcaacaaa acccttgtgg cggccaaatc gctgccgaaa
   720
   caggagagga tagagtggcc catggttgtg ctccgcgaga atggaaacat ctacattttg
   780
   atgaccggcg tggattcgga gaatacgcgc ctgcagggcc ccgttaccat aacgccacag
   840
   gcacatgaca attatggact ggagtcctgt gccctaatga ttatcccatc acttccgccg
   900
   accattgtta tagctgaatc caatggaaag cttcaccatg ctctgcttat ggaggcggag
   960
   gccactgagc attctttcaa tgaggttgac gactctgtgt tgattgaacc cgccgaatac
   1020
   gttgtacatg tactagaaac tgtcgaactt gagctgggaa tatctgcacc cgcaactgga
   1080
   aaagaaggag gcaactgtcc catatactta aagcgggatt tgatcaatga cgttcgctac
   1140
   tttgcctacc acaacgcagg acttcatgcc gttacggtca gctttatcgc agagctgcaa
   1200
   cgttacttgg aaagcgagtc cgatgaagat cgtctagagc tggcggtctc tgcaagtgca
   1260
   gagtacattc tgtgcacaaa attcgattcc agtgagacgg tgaacgcagt ctttggactt
   1320
   gccctattac aaattcctgc cggtatagtt ctactgctgg gcagcggtca ggtgattagc
   1380
   cttaagctgg taattgatgc ccagctgttg gttacaccta atgagaataa gccagttgac
   1440
   tcggaagtgt ctcagaagga aagtgggcca ccgtttgtcg atacgattaa atcgcttttg
   1500
   caacgtagtg ttaaccagcc catcttggcg gataaacttt cctcgccatc cgcccaggag
   1560
   agcttcgaac tgcttaacca ggcaatcgag gtgcttcgtg agcagtacct taaacgacat
   1620
   gaccttgtgc gcgccgcatt tacacgccac atcaaccaga tccagctgaa gaaggaacaa
   1680
   cagctgcagg agatccaaga cctggagcag gaacgcgaac tgatcagcga gcgggcccac
   1740
   aagcttgccg agcgcttcga ggagattagc tacaaccagg agctgctcgt ccgcaagtgc
   1800
   aacgctctga tgcagagggc caatgcctca ctacccaata gtgtgattgc agaacgggag
   1860
   ttttcgcagg aggtgattcg acttaataag gtcacccaga gcctggccgc tggactggag
   1920
   actgctaaga agacgtttaa caagcagcgc taccacattg cccagagtca agaggacctc
   1980
   aagaagaacg cttacgagct gccggagaaa cagcaccgca ctatcaccga gatacttacc
   2040
   cagctaaccg gggagatcga tcgccagatc accgacgtca agcgcattaa taaaatcgtt
   2100
   ggcatctaa
   2109
<210> 3
   <211> 702
   <212> PRT
   <213> Drosophila
<400> 3
   Met Ser Leu Thr Asp Val Leu Glu Leu Asn Lys Thr Glu Leu Phe Ala
   1               5                   10                  15
   Lys Ile Arg Asn Gly Leu Pro Val Val Gln Arg Thr Gln Asn Leu Leu
               20                  25                  30
   Asp Cys Lys Asp Asp Leu Leu Phe Ala Trp His Ala Lys Asp Ser Cys
           35                  40                  45
   Leu Leu Val Arg Asn Trp Arg Ser Ser Leu Ala Ala Lys Val Asn Ile
       50                  55                  60
   Gln Phe Gln Thr Leu Ile Pro Ser Ser Leu Val Ser Leu Glu Val Asp
   65                  70                  75                  80
   Arg Val Leu Ala Ser Asn Glu Gly Ser Leu Val Ala Leu Ser Gly Pro
                   85                  90                  95
   Arg Gly Val Val Ile Met Glu Leu Pro Arg Arg Trp Gly Pro Asp Gly
               100                 105                 110
   Tyr Tyr Lys Asp Gly Lys Pro Val Ile Thr Cys Arg Thr Phe Gly Leu
           115                 120                 125
   Asp Thr Gln Leu Phe Leu Lys Asn Pro His Leu Glu Val Arg Gln Val
       130                 135                 140
   Arg Trp His Pro Thr Ser Val Ser Asp Ser Thr Leu Leu Val Leu Leu
   145                 150                 155                 160
   Asn Asn Asn Thr Ile Arg Val Tyr Asn His Ser Lys Leu Arg His Val
                   165                 170                 175
   Trp Gln Val Gly Pro Pro Val Leu Arg Ser Gly Ala Asn Asn Ser Leu
               180                 185                 190
   Cys Asp Phe Gly Glu Leu Ala Val Asp Phe Asp Ile Ala Pro Ala Ala
           195                 200                 205
   Lys Pro Arg Val Thr Glu Pro Glu Thr Ala Gly Asn Asn Glu Thr Thr
       210                 215                 220
   Leu Asp Lys Ser Asn Lys Thr Leu Val Ala Ala Lys Ser Leu Pro Lys
   225                 230                 235                 240
   Gln Glu Arg Ile Glu Trp Pro Met Val Val Leu Arg Glu Asn Gly Asn
                   245                 250                 255
   Ile Tyr Ile Leu Met Thr Gly Val Asp Ser Glu Asn Thr Arg Leu Gln
               260                 265                 270
   Gly Pro Val Thr Ile Thr Pro Gln Ala His Asp Asn Tyr Gly Leu Glu
           275                 280                 285
   Ser Cys Ala Leu Met Ile Ile Pro Ser Leu Pro Pro Thr Ile Val Ile
       290                 295                 300
   Ala Glu Ser Asn Gly Lys Leu His His Ala Leu Leu Met Glu Ala Glu
   305                 310                 315                 320
   Ala Thr Glu His Ser Phe Asn Glu Val Asp Asp Ser Val Leu Ile Glu
                   325                 330                 335
   Pro Ala Glu Tyr Val Val His Val Leu Glu Thr Val Glu Leu Glu Leu
               340                 345                 350
   Gly Ile Ser Ala Pro Ala Thr Gly Lys Glu Gly Gly Asn Cys Pro Ile
           355                 360                 365
   Tyr Leu Lys Arg Asp Leu Ile Asn Asp Val Arg Tyr Phe Ala Tyr His
       370                 375                 380
   Asn Ala Gly Leu His Ala Val Thr Val Ser Phe Ile Ala Glu Leu Gln
   385                 390                 395                 400
   Arg Tyr Leu Glu Ser Glu Ser Asp Glu Asp Arg Leu Glu Leu Ala Val
                   405                 410                 415
   Ser Ala Ser Ala Glu Tyr Ile Leu Cys Thr Lys Phe Asp Ser Ser Glu
                   420                 425                 430
   Thr Val Asn Ala Val Phe Gly Leu Ala Leu Leu Gln Ile Pro Ala Gly
               435                 440                 445
   Ile Val Leu Leu Leu Gly Ser Gly Gln Val Ile Ser Leu Lys Leu Val
       450                 455                 460
   Ile Asp Ala Gln Leu Leu Val Thr Pro Asn Glu Asn Lys Pro Val Asp
   465                 470                 475                 480
   Ser Glu Val Ser Gln Lys Glu Ser Gly Pro Pro Phe Val Asp Thr Ile
                   485                 490                 495
   Lys Ser Leu Leu Gln Arg Ser Val Asn Gln Pro Ile Leu Ala Asp Lys
               500                 505                 510
   Leu Ser Ser Pro Ser Ala Gln Glu Ser Phe Glu Leu Leu Asn Gln Ala
           515                 520                 525
   Ile Glu Val Leu Arg Glu Gln Tyr Leu Lys Arg His Asp Leu Val Arg
       530                 535                 540
   Ala Ala Phe Thr Arg His Ile Asn Gln Ile Gln Leu Lys Lys Glu Gln
   545                 550                 555                 560
   Gln Leu Gln Glu Ile Gln Asp Leu Glu Gln Glu Arg Glu Leu Ile Ser
                   565                 570                 575
   Glu Arg Ala His Lys Leu Ala Glu Arg Phe Glu Glu Ile Ser Tyr Asn
               580                 585                 590
   Gln Glu Leu Leu Val Arg Lys Cys Asn Ala Leu Met Gln Arg Ala Asn
           595                 600                 605
   Ala Ser Leu Pro Asn Ser Val Ile Ala Glu Arg Glu Phe Ser Gln Glu
       610                 615                 620
   Val Ile Arg Leu Asn Lys Val Thr Gln Ser Leu Ala Ala Gly Leu Glu
   625                 630                 635                 640
   Thr Ala Lys Lys Thr Phe Asn Lys Gln Arg Tyr His Ile Ala Gln Ser
                   645                 650                 655
   Gln Glu Asp Leu Lys Lys Asn Ala Tyr Glu Leu Pro Glu Lys Gln His
               660                 665                 670
   Arg Thr Ile Thr Glu Ile Leu Thr Gln Leu Thr Gly Glu Ile Asp Arg
           675                 680                 685
   Gln Ile Thr Asp Val Lys Arg Ile Asn Lys Ile Val Gly Ile
       690                 695                 700
<210> 4
   <211> 741
   <212> PRT
   <213> Homo sapiens
<400> 4
   Met Ala Ala Ala Glu Gly Pro Val Gly Asp Gly Glu Leu Trp Gln Thr
    1               5                  10                  15
   Trp Leu Pro Asn His Val Val Phe Leu Arg Leu Arg Glu Gly Leu Lys
               20                  25                  30
   Asn Gln Ser Pro Thr Glu Ala Glu Lys Pro Ala Ser Ser Ser Leu Pro
           35                  40                  45
   Ser Ser Pro Pro Pro Gln Leu Leu Thr Arg Asn Val Val Phe Gly Leu
       50                  55                  60
   Gly Gly Glu Leu Phe Leu Trp Asp Gly Glu Asp Ser Ser Phe Leu Val
   65                  70                  75                  80
   Val Arg Leu Arg Gly Pro Ser Gly Gly Gly Glu Glu Pro Ala Leu Ser
                   85                  90                  95
   Gln Tyr Gln Arg Leu Leu Cys Ile Asn Pro Pro Leu Phe Glu Ile Tyr
               100                 105                 110
   Gln Val Leu Leu Ser Pro Thr Gln His His Val Ala Leu Ile Gly Ile
           115                 120                 125
   Lys Gly Leu Met Val Leu Glu Leu Pro Lys Arg Trp Gly Lys Asn Ser
       130                 135                 140
   Glu Phe Glu Gly Gly Lys Ser Thr Val Asn Cys Ser Thr Thr Pro Val
   145 150 155 160
   Ala Glu Arg Phe Phe Thr Ser Ser Thr Ser Leu Thr Leu Lys His Ala
   165 170 175
   Ala Trp Tyr Pro Ser Glu Ile Leu Asp Pro His Val Val Leu Leu Thr
   180 185 190
   Ser Asp Asn Val Ile Arg Ile Tyr Ser Leu Arg Glu Pro Gln Thr Pro
   195 200 205
   Thr Asn Val Ile Ile Leu Ser Glu Ala Glu Glu Glu Ser Leu Val Leu
   210 215 220
   Asn Lys Gly Arg Ala Tyr Thr Ala Ser Leu Gly Glu Thr Ala Val Ala
   225 230 235 240
   Phe Asp Phe Gly Pro Leu Asp Ala Val Pro Lys Thr Leu Phe Gly Gln
   245 250 255
   Asn Gly Lys Asp Glu Val Val Ala Tyr Pro Leu Tyr Ile Leu Tyr Glu
   260 265 270
   Asn Gly Glu Thr Phe Leu Thr Tyr Ile Ser Leu Leu His Ser Pro Gly
   275 280 285
   Asn Ile Trp Lys Ala Val Gly Ser Ile Ala His Ala Ser Ala Ala Glu
   290 295 300
   Asp Asn Tyr Gly Tyr Asp Ala Cys Ala Val Leu Cys Leu Pro Cys Val
   305 310 315 320
   Pro Asn Ile Leu Val Ile Ala Thr Glu Ser Gly Met Leu Tyr His Cys
   325 330 335
   Val Val Leu Glu Gly Glu Glu Glu Asp Asp His Thr Ser Glu Lys Ser
   340 345 350
   Trp Asp Ser Arg Ile Asp Leu Ile Pro Ser Leu Tyr Val Phe Glu Cys
   355 360 365
   Val Glu Leu Glu Leu Ala Leu Lys Leu Ala Ser Gly Glu Asp Asp Pro
   370 375 380
   Phe Asp Ser Asp Phe Ser Cys Pro Val Lys Leu His Arg Asp Pro Lys
   385 390 395 400
   Cys Pro Ser Arg Tyr His Cys Thr His Glu Ala Gly Val His Ser Val
   405 410 415
   Gly Leu Thr Trp Ile His Lys Leu His Lys Phe Leu Gly Ser Asp Glu
   420 425 430
   Glu Asp Lys Asp Ser Leu Gln Glu Leu Ser Thr Glu Gln Lys Cys Phe
   435 440 445
   Val Glu His Ile Leu Cys Thr Arg Pro Leu Pro Cys Arg Gln Pro Ala
   450 455 460
   Pro Ile Arg Gly Phe Trp Ile Val Pro Asp Ile Leu Gly Pro Thr Met
   465 470 475 480
   Ile Cys Ile Thr Ser Thr Tyr Glu Cys Leu Ile Trp Pro Leu Leu Ser
   485 490 495
   Thr Val His Pro Ala Ser Pro Pro Leu Leu Cys Thr Arg Glu Asp Val
   500 505 510
   Glu Val Ala Glu Ser Ser Leu Arg Val Leu Ala Glu Thr Pro Asp Ser
   515 520 525
   Phe Glu Lys His Ile Arg Ser Ile Leu Gln Arg Ser Val Ala Asn Pro
   530 535 540
   Ala Phe Leu Lys Ala Ser Glu Lys Asp Ile Ala Pro Pro Pro Glu Glu
   545 550 555 560
   Cys Leu Gln Leu Leu Ser Arg Ala Thr Gln Val Phe Arg Glu Gln Tyr
   565 570 575
   Ile Leu Lys Gln Asp Leu Ala Lys Glu Glu Ile Gln Arg Arg Val Lys
   580 585 590
   Leu Leu Cys Asp Gln Lys Lys Lys Gln Leu Glu Asp Leu Ser Tyr Cys
   595 600 605
   Arg Glu Glu Arg Lys Ser Leu Arg Glu Met Ala Glu Arg Leu Ala Asp
   610 615 620
   Lys Tyr Glu Glu Ala Lys Glu Lys Gln Glu Asp Ile Met Asn Arg Met
   625                 630                 635                 640
   Lys Lys Leu Leu His Ser Phe His Ser Glu Leu Pro Val Leu Ser Asp
                   645                 650                 655
   Ser Glu Arg Asp Met Lys Lys Glu Leu Gln Leu Ile Pro Asp Gln Leu
               660                 665                 670
   Arg His Leu Gly Asn Ala Ile Lys Gln Val Thr Met Lys Lys Asp Tyr
           675                 680                 685
   Gln Gln Gln Lys Met Glu Lys Val Leu Ser Leu Pro Lys Pro Thr Ile
       690                 695                 700
   Ile Leu Ser Ala Tyr Gln Arg Lys Cys Ile Gln Ser Ile Leu Lys Glu
   705                 710                 715                 720
   Glu Gly Glu His Ile Arg Glu Met Val Lys Gln Ile Asn Asp Ile Arg
                   725                 730                 735
   Asn His Val Asn Phe
               740
<210> 5
   <211> 742
   <212> PRT
   <213> Rattus norvegicus
<400> 5
   Met Ala Ala Ala Ala Gly Pro Val Gly Asp Gly Glu Leu Trp Gln Ser
   1 5 10 15
   Trp Leu Pro Asn His Val Val Phe Leu Arg Leu Arg Glu Gly Leu Lys
   20 25 30
   Asn Gln Ser Pro Ala Glu Ala Asp Lys Pro Ala Thr Ser Thr Ser Pro
   35 40 45
   Ser Cys Pro Pro Leu Pro Pro His Leu Pro Thr Arg Asn Leu Val Phe
   50 55 60
   Gly Leu Gly Gly Glu Leu Phe Leu Trp Asp Ala Glu Gly Ser Ala Phe
   65 70 75 80
   Leu Val Val Arg Leu Arg Gly Pro Ser Gly Gly Ser Val Glu Pro Pro
   85 90 95
   Leu Ser Gln Tyr Gln Arg Leu Leu Cys Ile Asn Pro Pro Leu Phe Glu
   100 105 110
   Ile His Gln Val Leu Leu Ser Pro Thr Gln His His Val Ala Leu Ile
   115 120 125
   Gly Thr Lys Gly Leu Met Ala Leu Glu Leu Pro Gln Arg Trp Gly Lys
   130 135 140
   Asp Ser Glu Phe Glu Gly Gly Lys Ala Thr Val Asn Cys Ser Thr Ile
   145 150 155 160
   Pro Ile Ala Glu Arg Phe Phe Thr Ser Ser Thr Ser Leu Thr Leu Lys
   165 170 175
   His Ala Ala Trp Tyr Pro Ser Glu Met Leu Asp Pro His Ile Val Leu
   180 185 190
   Leu Thr Ser Asp Asn Val Ile Arg Ile Tyr Ser Leu Arg Glu Pro Gln
   195 200 205
   Thr Pro Thr Lys Val Ile Val Leu Ser Glu Ala Glu Glu Glu Ser Leu
   210 215 220
   Ile Leu Asn Lys Gly Arg Ala Tyr Thr Ala Ser Leu Gly Glu Thr Ala
   225 230 235 240
   Val Ala Phe Asp Phe Gly Pro Leu Val Thr Val Ser Lys Asn Met Phe
   245 250 255
   Glu Gln Lys Asp Arg Glu Ala Val Ala Tyr Pro Leu Tyr Ile Leu Tyr
   260 265 270
   Glu Asn Gly Glu Thr Phe Leu Thr Tyr Val Ser Leu Leu His Ser Pro
   275 280 285
   Gly Asn Ile Gly Lys Leu Leu Gly Pro Leu Pro Met His Pro Ala Ala
   290 295 300
   Glu Asp Asn Tyr Gly Tyr Asp Ala Cys Ala Ile Leu Cys Leu Pro Cys
   305                 310                 315                 320
   Val Pro Asn Ile Leu Val Ile Ala Thr Glu Ser Gly Met Leu Tyr His
                   325                 330                 335
   Cys Val Val Leu Glu Gly Glu Glu Asp Asp Asp Gln Thr Leu Glu Lys
               340                 345                 350
   Ser Trp Asp Pro Arg Ala Asp Leu Ile Pro Ser Leu Tyr Val Phe Glu
           355                 360                 365
   Cys Val Glu Leu Glu Leu Ala Leu Lys Leu Ala Ser Ala Glu Asp Asp
       370                 375                 380
   Pro Phe Ala Ser Asp Phe Ser Cys Pro Ile Lys Leu His Arg Asp Pro
   385                 390                 395                 400
   Lys Cys Pro Ser Arg Tyr His Cys Ser His Glu Ala Gly Val His Ser
                   405                 410                 415
   Val Gly Leu Thr Trp Ile His Lys Leu His Lys Phe Leu Gly Ser Asp
               420                 425                 430
   Glu Glu Asp Lys Asp Ser Leu Gln Glu Leu Thr Ala Glu Gln Lys Cys
           435                 440                 445
   Phe Val Glu His Ile Leu Cys Thr Lys Pro Leu Pro Cys Arg Gln Pro
       450                 455                 460
   Ala Pro Ile Arg Gly Phe Trp Ile Val Pro Asp Ile Leu Gly Pro Thr
   465                 470                 475                 480
   Met Ile Cys Ile Thr Ser Thr Tyr Glu Cys Leu Ile Arg Pro Leu Leu
                   485                 490                 495
   Ser Thr Val His Pro Ala Ser Pro Pro Leu Leu Cys Thr Arg Glu Asp
               500                 505                 510
   Ala Gly Val Ala Glu Ser Pro Leu Arg Ile Leu Ala Glu Ala Pro Asp
           515                 520                 525
   Ser Phe Glu Lys His Ile Lys Arg Ile Leu Gln Arg Ser Ala Ala Asn
       530                 535                 540
   Pro Ala Leu Leu Lys Ser Ser Glu Lys Asp Leu Ala Pro Pro Pro Glu
   545                 550                 555                 560
   Glu Cys Leu Gln Leu Ile Ser Arg Ala Thr Gln Val Phe Arg Glu Gln
                   565                 570                 575
   Tyr Ile Leu Lys Gln Asp Leu Ala Lys Glu Glu Ile Gln Arg Arg Val
               580                 585                 590
   Lys Leu Leu Cys Asp Gln Lys Arg Lys Gln Leu Glu Asp Leu Asn Tyr
           595                 600                 605
   Cys Arg Glu Glu Arg Lys Ser Leu Arg Glu Met Ala Glu Arg Leu Ala
       610                 615                 620
   Asp Lys Tyr Glu Glu Ala Lys Glu Lys Gln Glu Asp Ile Met Asn Arg
   625                 630                 635                 640
   Met Lys Lys Val Leu His Ser Phe His Thr Gln Leu Pro Val Leu Ser
                   645                 650                 655
   Asp Ser Glu Arg Asp Met Lys Lys Glu Leu Gln Leu Ile Pro Asp Gln
               660                 665                 670
   Leu Arg His Leu Gly Asn Ala Ile Lys Gln Val Thr Met Lys Lys Asp
           675                 680                 685
   Tyr Gln Gln Arg Lys Met Glu Lys Val Leu Ser Pro Gln Lys Pro Thr
       690                 695                 700
   Ile Thr Leu Ser Ala Tyr Gln Arg Lys Cys Ile Gln Ser Ile Leu Lys
   705                 710                 715                 720
   Glu Glu Gly Glu His Ile Arg Glu Met Val Lys Gln Ile Asn Asp Ile
                   725                 730                 735
   Arg Asn His Val Asn Phe
               740

## Description

### Field of the Invention

The present invention relates to nucleic acid and amino acid sequences pertaining to nuclear receptors which selectively facilitate the entry of transcription factors into the cell nucleus and thereby regulate gene expression and cell/tissue physiology. The nuclear import biomolecules are specifically required for the import of Nuclear Factor-kappaB (NF-κB) and related transcription activators required for the immune response and the mediation of related pathophysiological disorders. Molecular sequences are provided for the design and synthesis of entities that modulate biological and/or pharmacological activity of native nuclear receptors. The sequences are also provided for employment to identify small molecule compounds that modulate biological and/or pharmacological activity of the native receptors. The invention is also drawn toward the study, prevention, diagnosis, and treatment of pathophysiological disorders related to the family of nuclear receptors.

### Background of the Invention

Developmental processes and physiological responses rely on signal transduction pathways that culminate with the selective nuclear entry of transcription factors and other regulatory proteins to control gene expression. *See,* e.g., Nigg, E. A., *Nucleocytoplasmic Transport: Signals, Mechanisms and Regulation*, Nature, *386*:779 (1997); Mattaj, I. W., *et al*., L. *Nucleocytoplasmic Transport: The Soluble Phase*, Annu. Rev. Biochem., 67:265 (1998). Protein import commences in the cytoplasm by the interaction between the protein that is targeted to the nucleus and its transport receptor via the nuclear localization signal (NLS), a short stretch of basic amino acids responsible for nuclear targeting. The transport receptor docks the complex to the nuclear pore and escorts the cargo through the pore. The difference between the various transport pathways is the use of distinct transport receptors.

The activation of an immune response relies to a large extent on the induced translocation of proteins from the cytoplasm into the nucleus. The Nuclear Factor-kappaB proteins (NF-κB), for example are involved in both *Drosophila* and mammalian responses to infection, and are held in the cytoplasm by interaction with an inhibitor protein, IκB. Upon signaling the IκB protein becomes phosphorylated and degraded, and the NF-κB protein is then free to move into the nucleus. *See,* e.g., Kopp, E. B., *et al*., Curr. Opin. Immunol., 11:13 (1999). In *Drosophila* three NF-κB proteins have been identified, *dorsal* (Steward, R., Dorsal, *An Emryonic Polarity gene in Drosophila, is Homologous to the Vertrbrate Proto*-*Oncogene*, *c-rel,* Science *238*, 692-694(1987)), *Dif* (Ip, Y. T., *et al*., *Dif, A Dorsal*-*Related Gene that Mediates an Immune Response in Drosophila,* Cell *75*, 753-763.(1997)), and *Relish* (Dushay, M. S., *et al*., *Origins of Immunity: Relish, a Compound Rel-Like Gene in the Antibacterial Defense of Drosophila,* Proc. Natl. Acad. Sci. USA, 93:10343 (1996)). The two former are retained in the cytoplasm by interaction with the IκB analogous cactus protein in *Drosophila*. Upon an immune response, these proteins are translocated into the nucleus. Kopp, E. B., *et al*., Curr. Opin. Immunol., 11:13 (1999). The NF-κB proteins are moreover key mammalian transcription factors which regulate, for example, the expression of pro-inflammatory genes. Despite the well defined signaling pathways that lead to activation of the NF-κB proteins, little has previously been known about the discriminating mechanism, namely the transport receptor, which facilitates selective translocation of NF-κB and related transcrimption factors into the nucleus.

The identifiction of a family of transport receptors which control the entry of NF-kB and related transcription factors into the cell nucleus, including mammalian cells, would convey an ability to specifically regulate gene expression and thereby facilitate broad therapeutic potential in the control of a myriad of pathophysiological disorders including but not limited to inflammation, asthma, rheumatoid arthritis (RA), chronic obstructive pulmonary disease (COPD), angiogenesis, septic shock, lung fibrosis, glomerulonephritis, artherosclerosis, AIDS, and apoptotic disorders.

### Summary of the Invention

The present invention is directed to an isolated and purified polynucleotide molecule comprising a nucleic acid sequence which encodes a polypeptide having at least about 90% homology to a member selected from (SEQ ID NO:3, positions 1-200).

Isolated and purified polynucleotides of the present invention include but are not limited to sequences which comprise SEQ ID NO:1 and/or SEQ ID NO:2.

The current invention is directed to a purified polypeptide selected from (SEQ ID NO:3, positions 1-200, or a variant thereof as defined herein.

The instant invention is further directed to methods of identifying compounds that modulate a biological and/or pharmacological activity of a transcription factor-selective nuclear transport receptor, comprising:
(a) combining a candidate compound modulator with a polypeptide comprising a sequence having at least about 90% homology to a member selected from (SEQ ID NO:3, positions 1-200), and
(b) measuring an effect of the candidate compound modulator on the biological and/or pharmacological activity of the polypeptide.

### Detailed Description of the Invention

Unless defined otherwise, all technical and scientific terms used herein have the same meaning as is commonly understood by one of skill in the art to which this invention belongs. All publications and patents referred to herein are incorporated by reference.

*Biological activity* as used herein in reference to a transcription factor-selective nuclear transport receptor polypeptide, e.g., SEQ ID NO:3, refers to the ability of the peptide to interact with and/or bind to the rel or NF-κB family of proteins, including but not limited to NF-κB, the NF-κB NLS motif, members of the *rel* protein family, *dorsal* (GENBANK access M23702), *Dif*, *relish,* DPSE-1 (GENBANK accession AF132561) and the Human homolog O00410, *shaq* proteins including but not limited to the product of human oncogene CAN 214 (CAN/Nup214) and Human homolog NM_005085.1, human CRM1 Y08614, STATs (e.g., STAT-1 (P42224), STAT-2 (NM005419), STAT-4 (L78440), STAT-5 (L41142), STAT-5B (U47686), and STAT-6 (NM003153), SMADs (e.g., Smad 6 (AFO35528 and AAB 94137), and Smad 3 (BAA22032 ), pRELB (GENBANK access NP_006500), KBF2 Human (GENBANK access Q00653) (NFkappa B p100), AAB21124 (NFkappa b p50 homolog), A42024 (NFkappa b p 100), A37867 (NFkappa b 50k precursor), AAB2343 7 (NFkappa b p105 homolog), CAA8052 (NFkappa b p65), pRAI NP_006654, pNFKBIE NP_004547, KBF1_human (GENBANK access P19838) (NFkappa b p105), CAA87683 (NFkappa b), RELB_human (GENBANK access Q01201) (RELB/I-REL), BCL3_human (GENBANK access P20749), AAA36408 (NFkappa b), and AAA36127 (I-REL). These are biological molecules whose nuclear entry is expected to be regulated by transcription factor-selective nuclear transport receptor polypeptides described herein. They are all implicated in the activation of the inflammatory response.

*Pharmacological activity* as used herein in reference to a transcription factor-selective nuclear transport receptor polypeptide, refers to the ability of the biomolecule to mediate cell processes related to the immune response and inflammation including but not limited to inflammation, asthma, rheumatoid arthritis (RA), chronic obstructive pulmonary disease (COPD), angiogenesis, septic shock, lung fibrosis, glomerulonephritis, artherosclerosis, AIDS, and apoptotic disorders; and/or the direct or indirect transcriptional activation of one or more genes. *See,* e.g., Chen F., *et al*., Clin. Chem., 45(1):7 (1999).

As depicted as used herein refers the sequence as well as inherent variants thereof, e.g., functional derivatives that demonstrate or perform substantially the same biological and/or pharmacological activity in substantially the same way. 'As depicted' is therefore intended to encompass variants of the subject molecule contemplated herein.

Variant as used herein refers to sequences substantiallly as shown having changes, e.g., a polypeptide sequence comprising a sequence which differs from the sequence referred to by at least one amino acid substitution, addition, or deletion, preferrably a conservative amino acid substitution, that demonstrate or perform substantially the same biological and/or pharmacological activity in substantially the same way, as well as truncated versions of these variants. However, variant as used herein is intended to encompass all *biologically effective dominant negative mutants.*

The term modulation is used herein to refer to the capacity to either enhance or inhibit a function of a biological molecule including, but not limited to, a biological and/or pharmacological activity of a transcription factor-selective nuclear transport receptor, or to the capacity to either enhance or inhibit a functional property of a nucleic acid regulatory or coding region. Modulate physiology as used herein refers to the biophysiological regulation of cells and/or tissue and the treatment of pathophysiological disorders related thereto.

Direct administration as used herein refers to the direct administration of nucleic acid molecules, peptides, or compounds as well as contemplated derivatives/variants of the present invention. Direct administration includes but is not limited to *ex vivo* as well as *in vivo* gene therapy techniques.

Purified as used herein refers to molecules, either nucleic acid or amino acid sequences, that are removed from their natural environment and isolated or separated from at least one other component with which they are naturally associated.

Expression vector as used herein refers to nucleic acid vector constructions to direct the transcription of nucleic acid regions in host cells. Expression vectors include but are not limited to plasmids, retroviral vectors, viral and synthetic vectors.

Transformed host cells as used herein refer to cells which harbor one or more nucleic acids of the present invention.

### Transcription Factor-Selective Nuclear Transport Receptors

Members Only *(mbo)* (SEQ ID NO:1), a *Drosophila* gene described herein, encodes a nuclear pore receptor (SEQ ID NO:3) that mediates import of dorsal NF-κB at the level of the nuclear pore and is required for activation of the Drosophila humoral immune response. The *mbo* gene product (SEQ ID NO:3) localizes to the nuclear envelope and is homologous to mammalian proteins including human Nup88 (SEQ ID NO:4) as well as rat Nup88 (SEQ ID NO:5) (also known as Nup84). Nup88 is reported to localize to the cytoplasmic filaments of the nuclear pores to provide binding sites for nuclear import substrates; however, the function of mammalian Nup88 has previously been unknown. Fomerod, M., *et al*., EMBO J. 16:807 (1997); Bastos, R., *et al*., J. Cell Biol. 137:989 (1997). The previously reported mammalian Nup88 molecules are herein identified and characterized as specific transport receptors which control the entry of NF-κB and related transcription factors into the cell nucleus

*Mbo* (SEQ ID NO:3) demonstrates extensive homology to Nup88 (SEQ ID NO:4) especially considering the evolutionary divergence of the species. Overall the amino acid position identity is 24% and the homology is 52% and extends throughout the length of each. The most striking feature of both Nup88 and *Mbo* is their C-terminal regions which have a high probability score of forming a coiled coil structure. Such structures are often found to mediate protein interactions. This region of human Nup88 is important for its association with the nuclear pore complex. Bastos, R., *et al*., J. Cell Biol. 137:989 (1997). The proteins also share enhanced sequence similarity within their N-terminal and central domains. Moreover, when human Nup88 is overexpressed in tissue-culture cells, the protein becomes distributed throughout the cytoplasm, but not in the nucleus. Similarly, in Drosophila embryos which overexpresses *Mbo* in tracheal cells using the UAS-GAL4 system, staining for *Mbo* reveals a uniform cytoplasmic distribution which demonstrates that *mbo* (SEQ ID NO:3) is normally localized on the cytoplasmic side of the nuclear envelope.

The immune response is an example of a process which to a large extent relies on the induced translocation of proteins from the cytoplasm into the cell nucleus. NF-kB transcription factors are involved *Drosophila* as well as mammalian responses to infection and are maintained in the cytoplasm. In addition to its role in the immune response, *Mbo* is dynamically expressed and required during several distinct developmental processes. During tracheal branch fusion, for example, *Mbo* appears to be involved in the morphogenetic process giving rise to a bicellular anastomosis connecting two independent branches. The nuclear translocation of the *Drosophila* NF-κB protein *dorsal* is induced to determine the dorso-ventral polarity of the early embryo. Morisato, D., *et al*., Ann. Rev. Gen. 29:371 (1995). *Dorsal* translocation is also part of the activation of the larval immune response to infection following the activation of the same signaling cascade. Lemaitre, B., *et al*., EMBO J., 14:536 (1995). Moreover, the *Drosophila* homologue of mammalian STATs is striking similar in its zygotic expression pattern and tracheal mutant phenotype to *Mbo*. STAT is also involved in the immune response of vertebrates, and is expected to have a similar role in *Drosophila.* Barillas-Mury, C., *et al*., EMBO J., 18:959 (1999). Thus, STATs appear, in addition to NF-κBs, to be targets for the family of transcription factor-selective nuclear transport receptors decribed herein (e.g., SEQ ID NO:3, SEQ ID NO:4, SEQ ID NO:5).

The subcellular localization of *dorsal* in homozygous *Mbo* larvae and their heterozygous siblings was investigated and is herein disclosed. Both mutant and heterozygous larvae where reared together, and in non-challenged larvae of both genotypes dorsal is demonstrated to be predominantly cytoplasmic. When the larvae were infected with a needle dipped in a bacterial culture, *dorsal* translocated into the nucleus within 45 minutes in the heterozygous larvae. *Mbo* mutant larvae that received the same treatment demonstrated no nuclear localization of *dorsal*. It is also demonstrated that *dorsal* becomes translocated in the lymph gland tissues in wild type larvae upon infection. Nuclear *dorsal* in lymph glands is not detected in unchallenged larvae; after challenge all larvae tested (n=100) display nuclear localization of *dorsal* in the lymph glands. The lymph glands of mutant larvae, however, show *dorsal* mainly in the cytoplasm after induction. Wild type larvae nuclear translocation of the rel proteins *dorsal* and *Dif* is followed by the rapid transcriptional activation of genes encoding antimicrobial peptides. Petersen, U. M., *et al*., EMBO J., 14:3146 (1995). A reporter construct of the inducible Cecropin A promoter coupled to LacZ (cec-lacZ) is strongly induced in wild type larvae upon infection; whereas it is non responsive in *Mbo* mutants. Further analysis of the inducible expression of the genes for antimicrobial peptides Drosomycin and Diptericin by Northern blot hybridizations revealed that their induction is severely impaired in *Mbo* mutant larvae. *Mbo* (SEQ ID NO:3) is required for the inducible translocation of the *Drosophila* NF-κB homologue *dorsal* into the nucleus and the concomitant transcriptional activation of the genes coding for antimicrobial peptides. Immunoprecipitations were carried out, using a polyclonal antiserum that has been shown before to immunoprecipitate *dorsal* from protein extracts, to demonstrate that *Mbo* directly interacts with *dorsal*. Gillespie, S. H., *et al*., Mol. Cell Biol., 14:3559 (1994). It is moreover demonstrated that *Mbo* is not required for general protein import and/or for mRNA export. Furthermore, *de novo* protein production can occur in *Mbo* mutants which demonstrates a fully functional basal transcription and translation machinery. Because the basic transport machinery across the nuclear pore is functional in *Mbo* mutants and since *in situ* antibody stainings with anti *Mbo* antiserum localize the protein in a punctuated ring around the nuclear envelope *Mbo* appears unequivocally to directly participate in the nuclear translocation of *dorsal.* Regulated nuclear import of the *Drosophila* NF-kB protein *dorsal* that normally occurs upon a immune response is prevented in *Mbo* mutants, despite the fact that upstream signaling events resulting in the degradation of the IkB protein *cactus* are intact. Moreover, as a result, downstream target genes which encode antimicrobial peptides are not activated. *Mbo* mutants do not show any defects in RNA export, classical nuclear localization signal (NLS)-dependent import or import of phosphorylated MAP kinase and cyclin B into the nucleus. Immunoprecipitations from embryonic extracts show complex formation between *Mbo* and *dorsal*.

### Pharmacological Significance

The identification of *Mbo* (SEQ ID NO:3) specifically required for the nuclear import of the Drosophila NF-kB protein *dorsal* and the corollary immune response, as well as the characterization of the pharmacological significance of mammlian homologues (SEQ ID NO:4, SEQ ID NO:5) presents valuable opportunities to control the nuclear import and therefore physiological effects of NF-kB (e.g., modulation of immune response) and related transcription factors.

Human *Shaq,* for example, was originally isolated as an oncogene where two different translocations affect the human gene and cause either a subtype of acute myeloid leukemia or acute undifferentiated leukemia. The mouse knockout of Shaq is lethal in utero and phenotypes include defects in nuclear RNA export whereas classical NLS-dependent import is not affected. A two hundred amino acid region in the coil domain of the Drosophila homologue of Shaq is identified as necessary for binding to the N-terminal 200 aa of *Mbo* (SEQ ID NO:3). It is the prediction that drugs targeting the interaction of these domains in mammalian *Mbo* homologues (e.g., SEQ ID NO:4, SEQ ID NO:5) will inhibit translocation of Rel-proteins. Data also indicate that certain STAT factors require *Mbo* for their translocation.

The transcription factor-selective nuclear transport receptors, particularly mammalian *Mbo* homologues and variations contemplated herein, provide new targets for drugs to specifically regulate gene expression and thereby facilitates broad therapeutic potential in the control of a myriad of pathophysiological disorders including but not limited to inflammation, asthma, rheumatoid arthritis (RA), chronic obstructive pulmonary disease (COPD), angiogenesis, septic shock, lung fibrosis, glomerulonephritis, atherosclerosis, AIDS, and apoptotic disorders. Accordingly, the ability to modulate a biological and/or pharmacological activity of a transcription factor-selective nuclear transport receptor polypeptide is expected to confer the ability to ameliorate these pathophysiological conditions. The human homologue (SEQ ID NO:4) and other homologues (e.g., SEQ ID NO:5) of the nuclear import protein *Mbo* in Drosophila (SEQ ID NO:3) are valuable targets for selective drug intervention of transcription factor, e.g., NF-kB, entry into the nucleus.

The present invention therefore relates to methods of identifying molecules capable of modulating a biological and/or pharmacological activity of a transcription factor-selective nuclear transport receptor polypeptide, e.g., SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725. Polynucleotide sequences which encode these molecules and variants thereof contemplated herein are particularly preferred embodiment of the present invention.

### Variants

The present invention relates to variants of nucleic acid sequences sequences and amino acid sequences substantially as shown or known, which have changes, e.g., a polypeptide sequence comprising a sequence which differs from the sequence referred to by at least one amino acid substitution, preferrably a conservative amino acid substitution (or a nucleic acid sequence which encodes therefor), that demonstrate or perform substantially the same biological and/or pharmacological activity in substantially the same way, as well as molecules which comprise truncated versions. However, variant as used herein is intended to encompass *biologically effective dominant negative mutants.*

Preferred variants of molecules described herein, for example, comprise an amino acid sequences having at least 90% amino acid sequence homology (identity) to a sequence selected from SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725 or a biologically and/or pharmacologically active substantial fragment thereof

A variant of a transcription factor-selective nuclear transport receptor polypeptide may have an amino acid sequence that is different by one or more amino acid substitutions. Embodiments which comprise amino acid deletions and/or additions are also contemplated. The variant may have conservative changes (amino acid similarity), wherein a substituted amino acid has similar structural or chemical properties, for example, the replacement of leucine with isoleucine. A variant may have nonconservative changes, e.g., replacement of a glycine with a tryptophan. Guidance in determining which and how many amino acid residues may be substituted, inserted or deleted without abolishing biological or proposed pharmacological activity may be reasonably inferred in view of this disclosure and may be further be found using computer programs well known in the art, for example, DNAStar software.

Amino acid substitutions may be made, for instance, on the basis of similarity in polarity, charge, solubility, hydrophobicity, hydrophilicity, and/or the amphipathic nature of the residues as long as a biological and/or pharmacological activity of the native molecule is retained.

Negatively charged amino acids, for example, include aspartic acid and glutamic acid; positively charged amino acids include lysine and arginine; and amino acids with uncharged polar head groups having similar hydrophilicity values include leucine, isoleucine, valine; glycine, alanine; asparagine, glutamine; serine, threonine phenylalanine, and tyrosine. However, in the construction of biologically effective dominant negative mutants at least one amino acid residue position at an active site required for biological and/or pharmacological activity in the native peptide is changed to produce an agent or entity having reduced activity or which is devoid of detectable activity.

Suitable substitutions of amino acids include the use of a chemically derivatized residue in place of a non-derivatized residue. D-isomers as well as other known derivatives may also be substituted for the naturally occurring amino acids. *See, e.g.,* U.S. Patent No. 5,652,369, *Amino Acid Derivatives*, issued July 29, 1997. Example substitutions are set forth in TABLE 1 as follows:

**Table 1**

| **Original residue** | **Example conservative substitutions** |
|---|---|
| Ala (A) | Gly; Ser; Val; Leu; Ile; Pro |
| Arg (R) | Lys; His; Gin; Asn |
| Asn (N) | Gln; His; Lys; Arg |
| Asp (D) | Glu |
| Cys (C) | Ser |
| Gln (Q) | Asn |
| Glu (E) | Asp |
| Gly (G) | Ala; Pro |
| His (H) | Asn; Gln; Arg; Lys |
| Ile (I) | Leu; Val; Met; Ala; Phe |
| Leu (L) | Ile; Val; Met; Ala; Phe |
| Lys (K) | Arg; Gln; His; Asn |
| Met (M) | Leu; Tyr; Ile; Phe |
| Phe (F) | Met; Leu; Tyr; Val; Ile; Ala |
| Pro (P) | Ala; Gly |
| Ser (S) | Thr |
| Thr (T) | Ser |
| Trp (W) | Tyr; Phe |
| Tyr (Y) | Trp; Phe; Thr; Ser |
| Val (V) | Ile; Leu; Met; Phe; Ala |

"Homology" as used in this description is a measure of the *identity* of nucleotide sequences or amino acid sequences. In order to characterize the homology, subject sequences are aligned so that the highest order homology (match) is obtained. "Identity" *per se* has an art-recognized meaning and can be calculated using published techniques. Computer program methods to determine identity between two sequences, for example, include DNAStar software (DNAStar Inc., Madison, WI); the GCG program package (Devereux, J., *et al*., Nucleic Acids Research (1984) 12(1):387); BLASTP, BLASTN, FASTA (Atschul, S. F. *et al*., J Molec Biol (1990) 215:403). Homology (identity) as defined herein is determined conventionally using the well known computer program, BESTFIT (Wisconsin Sequence Analysis Package, Version 8 for Unix, Genetics Computer Group, University Research Park, 575 Science Drive, Madison, Wis., 53711). When using BESTFIT or any other sequence alignment program to determine whether a particular sequence is, for example, about 90% homologous to a reference sequence, according to the present invention, the parameters are set such that the percentage of identity is calculated over the full length of the reference nucleotide sequence or amino acid sequence and that gaps in homology of up to about 10% of the total number of nucleotides in the reference sequence are allowed. Eighty percent of homology is therefore determined, for example, using the BESTFIT program with parameters set such that the percentage of identity is calculated over the full length of the reference sequence, e.g., SEQ ID NO:3, and gaps of up to 10% of the total number of amino acids in the reference sequence are allowed, and wherein up to 10% of the amino acid residues in the reference sequence may be deleted or substituted with another amino acid, or a number of amino acids up to 10% of the total amino acid residues in the reference sequence may be inserted into the reference sequence. Percent homologies are likewise determined, for example, to identify preferred species, within the scope of the claims appended hereto, which reside within the range of about 90 percent to 100 percent homology to SEQ ID NO:3 as well as biologically and/or pharmacologically active derivatives thereof.

Percentage *similarity* (conservative substitutions) between two polypeptides may also be scored by comparing the amino acid sequences of the two polypeptides by using programs well known in the art, including the BESTFIT program, by employing default settings for determining similarity.

Transcription factor-selective nuclear transport receptor molecules described herein, e.g., SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725, as well as nucleic acid molecules which comprise a sequence which encodes therefor, are used in screening assays to identify antagonists or inhibitors which bind to, or interact with the nuclear receptor molecules, emulate its substrate, or otherwise inactivate one of the biomolecule or compete biologically, e.g., competitive interaction or competitive binding inhibition. The nuclear receptor molecules are also used in screening assays to identify agonists which agonize or mimic the biological and/or pharmacological activity, induce the production of or prolong the biological halflife of the molecule *in vivo* or *in vitro*.

### Generally Acceptable Vectors

In accordance with the present invention, polynucleotide sequences which encode a nuclear transport receptor described herein or a corresponding polypeptide, dominant negative mutant versions, fusion proteins, or antisense molecules may be used in recombinant DNA molecules that direct the expression of the respective molecule in appropriate host cells. Due to the inherent degeneracy of the genetic code, other DNA sequences which encode substantially the same or a functionally equivalent amino acid sequence may be used to clone and express the transcription factor-selective nuclear transport receptor polypeptide, as well as variations thereto and dominant negative mutants thereof. As will be understood by those of skill in the art, it may be advantageous to produce nucleotide sequences possessing non-naturally occurring codons.

SEQ ID NO:2, for example, may be recombinantly expressed by molecular cloning into an expression vector containing a suitable promoter and other appropriate transcription regulatory elements, and transferred into prokaryotic or eukaryotic host cells to produce the native biomolecule. Techniques for such manipulations are fully described in Sambrook, J., *et al*., Molecular Cloning Second Edition, Cold Spring Harbor Press (1990), and are well known in the art.

Expression vectors are described herein as nucleic acid sequences for the transcription of embodiments of the present invention. Such vectors can be used to express nucleic acid sequences in a variety of hosts such as bacteria, bluegreen algae, plant cells, insect cells, fungal cells, human, and animal cells. Specifically designed vectors allow the shuttling of DNA between hosts such as bacteria-yeast, or bacteria-animal cells, or bacteria-fungal cells, or bacteria-invertebrate cells.

A variety of mammalian expression vectors may be used to express nuclear transport receptor molecules as well as variants and derivatives contemplated herein. Commercially available mammalian expression vectors which are suitable for recombinant expression, include but are not limited to, pcDNA3 (Invitrogen), pMCIneo (Stratagene), pXT1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and 1ZD35 (ATCC 37565), pLXIN and pSIR (CLONTECH), pIRES-EGFP (CLONTECH). INVITROGEN corporation provides a wide variety of commercially available mammalian expression vector/systems which can be effectively used with the present invention. INVITROGEN, Carlsbad, CA. *See*, *also*, PHARMINGEN products, vectors and systems, San Diego, CA.

Baculoviral expression systems may also be used with the present invention to produce high yields of biologically acive transcription factor-selective nuclear transport receptor. Vectors such as the CLONETECH, BacPak™ Baculovirus expression system and protocols are preferred which are commercially available. CLONTECH, Palo Alto, CA. Miller, L.K., *et al*., Curr. Op. Genet. Dev. 3:97 (1993); O'Reilly, D.R, *et al*., *Baculovirus Expression Vectors: A Laboratory Manual*, 127. Vectors such as the INVITROGEN, MaxBac™ Baculovirus expression system, insect cells, and protocols are also preferred which are commercially available. INVITROGEN, Carlsbad, CA.

### Example Host Cells

Host cells transformed with a nucleotide sequence which encodes the nuclear transport receptor mlecules described herein may be cultured under conditions suitable for the expression and recovery of the encoded protein from cell culture. Preferred embodiments for use in methods of the invention are host cells transformed with a purified polynucleotide comprising a nucleic acid sequence which encodes a polypeptide comprising a sequence as depicted in SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and/or SEQ ID NO:5 positions 575-725 or a contemplated homolog or variant thereof. Cells of this type or preparations made from them may be used to screen for modulators of the biological and/or pharmacological activity of the recited molecules.

Eukaryotic recombinant host cells are especially preferred. Examples include but are not limited to yeast, mammalian cells including but not limited to cell lines of human, bovine, porcine, monkey and rodent origin, and insect cells including but not limited to Drosophila and silkworm derived cell lines. Cell lines derived from mammalian species which may be suitable and which are commercially available, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616),BS-C-1 (ATCC CCL 26) and MRC-5 (ATCC CCL 171).

The expression vector may be introduced into host cells to express a transcription factor-selective nuclear transport receptor polypeptide and derivatives thereof *via* any one of a number of techniques including but not limited to transformation, transfection, lipofection, protoplast fusion, and electroporation. Commercially available kits applicable for use with the present invention for hererologous expression, including well-characterized vectors, transfection reagents and conditions, and cell culture materials are well-established and readily available. CLONTECH, Palo Alto, CA; INVITROGEN, Carlsbad, CA; PHARMINGEN, San Diego, CA; STRATAGENE, LaJolla, CA. The expression vector-containing cells are clonally propagated and individually analyzed to determine the level of transcription factor-selective nuclear transport receptor polypeptide production. Identification of host cell clones which express a transcription factor-selective nuclear transport receptor polypeptide may be performed by several means, including but not limited to immunological reactivity with antibodies described herein, and/or the presence of host cell-associated specific biological activity, and/or the ability to covalently cross-link specific substrate to a transcription factor-selective nuclear transport receptor polypeptide with the bifunctional cross-linking reagent disuccinimidyl suberate or similar cross-linking reagents.

Transcription factor-selective nuclear transport receptor polypeptides may be expressed to facilitate practicing methods of the invention as a recombinant protein with one or more additional polypeptide domains added to facilitate protein purification. Such purification facilitating domains include, but are not limited to, metal chelating peptides such as histidine-tryptophan modules that allow purification on immobilized metals (Porath, J., Protein Exp. Purif. 3:263 (1992)), protein A domains that allow purification on immobilized immunoglobulin, and the domain utilized in the FLAGS extension/affinity purification system (Immunex Corp, Seattle WA). The inclusion of a cleavable linker sequences such as Factor XA or enterokinase (Invitrogen, San Diego CA) between the purification domain and the coding region is useful to facilitate purification.

Systems such as the CLONTECH, TALON™ nondenaturing protein purification kit for purifying 6xHis-tagged proteins under native conditions and protocols are preferred which are commercially available. CLONTECH, Palo Alto, CA.

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation and acylation. Post-translational processing which cleaves a nascent form of the protein may also be important for correct insertion, folding and/or function. Different host cells such as CHO, HeLa, MDCK, 293, WI38, NIH-3T3, HEK293 etc., have specific cellular machinery and characteristic mechanisms for such post-translational activities and may be chosen to ensure the correct modification and processing of the introduced, foreign protein.

For long-term, high-yield production of recombinant proteins, stable expression is preferred. For example, cell lines which stably express SEQ ID NO:4, for example, may be transformed using expression vectors which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clumps of stably transformed cells can be proliferated using tissue culture techniques appropriate to the cell type.

The nuclear transport receptor peptides can be produced in the yeast *S.cerevisiae* following the insertion of the optimal cDNA cistron into expression vectors designed to direct the intracellular or extracellular expression of the heterologous protein. In the case of intracellular expression, vectors such as EmBLyex4 or the like are ligated to the beta subunit cistron. *See, e.g.,* Rinas, U., *et al*., Biotechnology, 8:543 (1990); Horowitz, B., *et al*., J. Biol. Chem., 265:4189 (1989). For extracellular expression, the receptor 'cistron' is ligated into yeast expression vectors which may employ any of a series of well-characterized secretion signals.

A variety of protocols for detecting and measuring the expression of the novel molecule as well as functional derivatives thereof, using either polyclonal or monoclonal antibodies specific for the protein are known in the art. See Example I. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two non-interfering epitopes may be employed. Well known competitive binding techniques may also be employed. See, e.g., Hampton, R., *et al*. (1990), *Serological Methods - a Laboratory Manual*, APS Press, St Paul Minn.; Maddox, D.E., *et al*., J. Exp. Med. 158:1211.

### Example Embodiments

A purified polynucleotide is preferred which comprises a nucleic acid sequence which encodes a polypeptide having at least about 90% homology to a member selected from (SEQ ID NO:3, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725).

Methods are provided to screen compounds individually, or libraries of compounds, for the identification of compounds which have the ability to modulate a biological and/or pharmacological activity of a transcription factor-selective nuclear transport receptor polypeptide, particularly SEQ ID NO:4 and/or SEQ ID NO:5. The present invention is also directed to methods of screening for compounds which modulate the expression (transcription and/or translation) of DNA or RNA which encode transcription factor-selective nuclear transport receptors. Compounds which modulate these activities may be DNA, RNA, peptides, proteins, or non-proteinaceous organic molecules (e.g., small molecule compounds).

Example assays described herein are used to identify agents which modulate nuclear transport receptor biological interaction with NF-κB family peptides and/or a pharmacological activity of a transcription factor-selective nuclear transport receptor polypeptide. Such modulating agents are contemplated for therapeutic purposes, i.e., administration for the treatment of pathophysiological disorders related to the biological and/or pharmacological activity of a transcription factor-selective nuclear transport receptor, e.g., SEQ ID NO:4.

Compounds may modulate an ultimate biological and/or pharmacological activity by increasing or attenuating the expression of DNA or RNA encoding a transcription factor-selective nuclear transport receptor. Compounds that modulate the expression of DNA or RNA encoding such a receptor or the function of the polypeptide may be detected by a variety of assays. The assay may be a simple "yes/no" assay to determine whether there is a change in expression or activity. The assay may be made quantitative by comparing the expression or function of a test sample with the levels of expression or function in a standard sample.

The transcription factor-selective nuclear transport receptor polypeptides as depicted as well as contemplated variants can be used for screening therapeutic compounds in any of a variety of drug screening techniques. The fragment or entity employed in such a test may be free in solution, affixed to a solid support, borne on a cell surface, or located intracellularly. The abolition or modulation of activity or the formation of binding complexes, between a nuclear transport receptor molecule and the agent being tested, may be measured, for example, by means provided (see, Examples appended hereto). Accordingly, the present invention provides a method for screening a plurality of compounds for specific binding affinity with a polypeptide, e.g., SEQ ID NO:4, or a variant thereof contemplated herein, comprising providing a plurality of compounds; combining an example nuclear transport receptor polypeptide with each of a plurality of compounds for a time sufficient to allow binding under suitable conditions; and detecting binding of an embodiment of a nuclear transport receptor, to each of the plurality of compounds, thereby identifying the compounds which specifically bind the transport receptor polypeptide. Disruption of SEQ ID NO:4 binding to NF-κB, for example, is readily measurable by means of competitive binding assays well known in the art.

Methods of identifying compounds that modulate a biological and/or pharmacological activity of a transcription factor-selective nuclear transport receptor polypeptide, e.g., SEQ ID NO:4, are generally preferred, which comprise combining a candidate compound modulator with a purified polypeptide comprising the amino acid sequence as depicted in SEQ ID NO:4 or a variant of SEQ ID NO:4 having at least about 90% homology to a member selected from (SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725), and measuring an effect of the candidate compound modulator on the biological and/or pharmacological activity of the polypeptide. Preferred assays for modulators of nuclear transport receptors described herein fall into two general categories: 1) direct measurement of a biological activity as defined herein; and, 2) measurement of downstream events in the signaling cascade including cell/tissue/organism physiological manifestations.

In another embodiment of the invention to identify agents which modulate a biological activity of the biomolecules recited herein, a nucleic acid sequence which encodes a transcription factor-selective nuclear transport receptor polypeptide as depicted may be ligated to a heterologous sequence to encode a fusion protein for use in a yeast 2-hybrid system. To screen compounds for the modulation of biological activity, it is necessary to encode a chimeric molecule as described herein for expression in hererologous host cells. Chimeric constructs are also used to express a 'bait', according to methods well known using a yeast two-hybrid system, using accessory native peptides that are expected to be associated with a nuclear transport receptor, e.g., SEQ ID NO:4. The two-hybrid system uses the ability of a pair of interacting proteins to bring a transcription activation domain into close proximity with a DNA-binding site that regulates the expression of an adjacent reporter gene. Compounds which are able to modulate the biological activity of a biomolecule as defined herein are identified by the their ability to effect protein: protein interactions (reconstitution of the chimeric transcriptional activators) and hence the yeast 2-hybrid readout assays well-known to artisans of ordinary skill in this area of molecular biology. Fields, S., *et al*., Trends Genet., 10:286 (1994); Allen, J.B., *et al*., TIBS, 20:511 (1995). Fields, S., Song, O., Nature 340:245 (1989). Commercially available systems such as the CLONTECH, Matchmaker™ systems and protocols may be used with the present invention. CLONTECH, Palo Alto, CA. See also, Mendelsohn, A.R., Brent, R., Curr. Op. Biotech., 5:482 (1994); Phizicky. E.M., Fields, S., Microbiological Rev., 59(1):94 (1995); Yang, M., et al., Nucleic Acids Res., 23(7):1152 (1995); Fields, S., Sternglanz, R., TIG, 10(8):286 (1994); and US Patents 5,283,173, *System to Detect Protein-Protein Interactions*, and 5,468,614, which are incorporated herein by reference.

Compounds which are identified generally according to methods described, contemplated, and referenced herein that modulate a biological and/or pharmacological activity of a transcription factor-selective nuclear transport receptor polypeptide, e.g., the sequence as depicted in SEQ ID NO:4 and/or SEQ ID NO:5, are especially preferred embodiments of the present invention.

An especially preferred embodiment of the present invention is a method for treatment of a patient in need of such treatment for a dysfunctional- transcription factor-selective nuclear transport receptor related condition which is mediated by a human transcription factor-selective nuclear transport receptor, e.g., SEQ ID NO:4, comprising administration of a therapeutically effective amount of a modulating compound which was identified by means of a method of identifying compounds that modulate a biological and/or pharmacological activity of a transcription factor-selective nuclear transport receptor, comprising combining a candidate compound modulator with an isolated and purified polynucleotide molecule comprising a nucleic acid sequence which encodes a polypeptide comprising an amino acid sequence selected from (SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725), and measuring an effect of the candidate compound modulator on the biological and/or pharmacological activity of the corresponding polypeptide.

A method of modulating a biological and/or pharmacological activity of a transcription factor-selective nuclear transport receptor polypeptide in a cell is preferred which comprises administering an effective amount of a polynucleotide comprising a sequence which encodes a polypeptide comprising a sequence as depicted by (SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, and SEQ ID NO:5 positions 575-725), to said cell.

A method of modulating a biological and/or pharmacological activity of a transcription factor-selective nuclear transport receptor in a cell, tissue, or organism is preferred which comprises administering an effective amount of a polynucleotide comprising a nucleic acid sequence derived from a nucleic acid which encodes SEQ ID NO:3, SEQ ID NO:4, SEQ ID NO:4 positions 1-200, SEQ ID NO:4 positions 1-400, SEQ ID NO:4 positions 50-350, SEQ ID NO:4 positions 550-740, SEQ ID NO:4 positions 575-725, SEQ ID NO:5, SEQ ID NO:5 positions 1-200, SEQ ID NO:5 positions 1-400, SEQ ID NO:5 positions 50-350, SEQ ID NO:5 positions 550-740, or SEQ ID NO:5 positions 575-725 including but not limited to biologically-effective antisense molecules derived therefrom as well as dominant negative mutant versions.

### Compositions

Pharmaceutically useful therapeutic compositions which comprise a nucleic acid contemplated herein, or a compound identified by means encompassed by the claims appended hereto that modulates the biological and/or pharmacological activity of a transcription factor-selective nuclear transport receptor, e.g., SEQ ID NO:4, may be formulated according to known methods such as by the admixture of a pharmaceutically acceptable carrier. Examples of such carriers and methods of formulation may be found in *Remington's Pharmaceutical Sciences* (Maack Publishing Co, Easton, PA). To form a pharmaceutically acceptable composition suitable for effective administration, such compositions will contain an effective amount of a polypeptide, nucleic acid, or compound modulator.

Therapeutic compositions of the invention are administered to an individual or used in amounts sufficient to treat or diagnose transcription factor-selective nuclear transport receptor-related disorders. The effective amount may vary according to a variety of factors such as the individual's condition, weight, sex and age. Other factors include the mode of administration.

Pharmaceutical compositions suitable for use in the present invention include compositions wherein the active ingredients are contained in an effective amount to achieve the intended purpose. The determination of an effective dose is well within the capability of those skilled in the art. The therapeutically effective dose can be estimated initially either in cell culture assays, eg, of neoplastic cells, or in animal models, usually mice, rabbits, dogs, or pigs. The animal model is also used to achieve a desirable concentration range and route of administration. Such information can then be used to determine useful doses and routes for administration in humans. A therapeutically effective dose refers to that amount of compound, peptide, or nucleic acid which ameliorate or prevent a dysfunctional apoptotic condition. The exact dosage is chosen by the individual physician in view of the patient to be treated.

Compounds identified according to the methods disclosed herein as well as therapeutic nucleic acids and peptides contemplated herein may be used alone at appropriate dosages defined by routine testing in order to obtain optimal modulation of transcription factor-selective nuclear transport receptor activity. In addition, coadministration or sequential administration of these and other agents may be desirable.

The pharmaceutical compositions may be provided to the individual by a variety of routes such as subcutaneous, topical, oral and intramuscular. Administration of pharmaceutical compositions is accomplished orally or parenterally. Methods of parenteral delivery include topical, intra-arterial (directly to the tissue), intramuscular, subcutaneous, intramedullary, intrathecal, intraventricular, intravenous, intraperitoneal, or intranasal administration. The present invention also has the objective of providing suitable topical, oral, systemic and parenteral pharmaceutical formulations for use in the novel methods of treatment of the present invention. The compositions containing compounds identified according to this invention as the active ingredient for use in the modulation of a transcription factor-selective nuclear transport receptor can be administered in a wide variety of therapeutic dosage forms in conventional vehicles for administration. For example, the compounds can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound, nucleic acid, or peptide desired can be employed as an the transcription factor-selective nuclear transport receptor modulating agent.

The daily dosage of the products may be varied over a wide range from 0.01 to 1,000 mg per adult human/per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. Even more particularly, the range varies from about 0.05 to about 1 mg/kg. Of course the dosage level will vary depending upon the potency of the particular compound. Certain compounds will be more potent than others. In addition, the dosage level will vary depending upon the bioavailability of the compound. The more bioavailable and potent the compound, the less compound will need to be administered through any delivery route, including but not limited to oral delivery. The dosages of transcription factor-selective nuclear transport receptor modulators are adjusted when combined to achieve desired effects. On the other hand, dosages of these various agents may be independently optimized and combined to achieve a synergistic result wherein the pathology is reduced more than it would be if either agent were used alone. Those skilled in the art will employ different formulations for nucleotides than for proteins or their inhibitors. Similarly, delivery of polynucleotides or polypeptides will be specific to particular cells and conditions.

### Examples

### Example I: Antibodies

To prepare the *Mbo* antiserum, a DNA fragment from the *mbo* cDNA (SEQ ID NO:1) (generated using HincII and KpnI) encoding SEQ ID NO:3 amino acids 505 to 702 was inserted in frame to the 6xHis coding sequence in pRSET C (cut with PvuII and KpnI; Invitrogen). The histidine-tagged *Mbo* fusion-protein was expressed in *E*. *coli,* purified on Ni-NTA resin (QIAGEN), and used for immunisation of rabbits at Agrisera (Umeå, Sweden). The antiserum was affinity purified on Ni-NTA resin coupled with the histidine-tagged *Mbo* fusion protein and used at 1:100 dilution for Western blots and immunohistochemistry. Fixation was reduced to 15 minutes and the washes were shortened to 2x5 minutes followed by 2x30 minutes.

### Example II: Analysis of Immune Response

To prepare larvae with low background levels of dorsal translocation, *Drosophila* eggs were collected on apple plates, dechorionated and transferred to new apple plates where they developed into larvae. The larvae fed on reconstituted dry yeast and were regularly transferred to new plates. For each experiment, larvae from the same plate were used. Injury was performed on third instar larvae by pricking the posterior part with a tungsten needle dipped in a mixed concentrated culture of *E*. *coli* and *M*. *luteus.* The infected larvae were analysed 45 min to 1 hour later for dorsal translocation, 2 hours later for antimicrobial peptide expression and 30 minutes later for cactus degradation. Dorsal translocation was analysed by antibody staining as described. Cactus degradation was assayed on western blots; whole larvae were turned inside-out, the gut was removed and the larval tissue transferred to sample buffer. After freezing and boiling, the samples were subjected to SDS-PAGE. Electrophoresis and immunoblotting were performed according to Sambrook et al. (1989). The polyclonal cactus antiserum (from S. Wassermann; Reach et al 1996, dev. biol 180) was used at 1/1000 dilution. Hybridising bands were detected with the Amersham ECL reagent according to the manufacturer. Antibacterial peptide induction was assayed by cecropin-LacZ expression using anti-beta galactosidase antibodies and by Northern blots of total RNA.

### Example III: High Throughput Screening - Scintillation Proximity Assay (SPA)

A high throughput screening assay is set up using *Mbo,* e.g., SEQ ID NO:3, expressed in either insect cells using the baculovirus expression system, or other mammalian cell lines such as CHO, Cos, or MEL. See, *e*.*g*., Needham M, *et al*., Protein Expr. Purif., 7(2):173 (1996); Life Technologies, Gaithersburg, MD. Bac-to-Bac™ HT Baculovirus Expression System. *Mbo* can be tagged *via* expression as a fusion protein from commercially available expression vectors containing sequences, for example, FLAG, or c-myc/6-His to aid in purification and detection as is well-known in the art. Materials, purification and detection methods for FLAG-tag are available, for example, from Kodak, New Haven CT. Vectors, for use in the assay, include pFLAG.MAC for N-terminal expression of FLAG or pFLAG.CTC for C-terminal expression of FLAG (Kodak, New Haven CT). Purification method uses anti-FLAG M2 Affinity Gel. Detection is performed using monoclonal antibody Anti-FLAG M2 Monoclonal Antibody (# IB13010) (Kodak, New Haven CT). C-myc/6-His-tag vectors, methods and detection reagents are obtained from Invitrogen Carlsbad CA: vector pcDNA3.1(-)/Myc-His Xpress™; and, purification: Xpress™ Purification System; and, detection: Anti-myc Antibody (# R950-25) or Anti-His(C-term) Antibody (# R930-25).

After expression and purification of the tagged *Mbo* the purified protein is fluorescently labeled through conjugation to BODIPY FL (Molecular Probes Eugene, OR) per the protocol provided by the manufacturer.

Membranes of cells expressing *Mbo* are dissociated to release the protein by resuspending the membranes in 0.1 - 1% Triton X-100. Excess lipids are pelleted via centrifugation. Luo H., et al., Biochim Biophys Acta, 1052:119 (1990); Reyl-Desmars F., et al., J.B.C., 264:18789 (1989). Solubilized protein is bound to polylysine coated SPA beads (Amersham, Arlington Heights, IL) per the manufacturer's protocol.

BODIPY FL-labeled *Mbo* is incubated alone and separately in the presence of candidate modulators with the protein-bound SPA beads. The beads are washed free of unbound BODIPY-*Mbo*. The relative amount of bound BODIPY-*Mbo* in each candidate sample is determined by measuring fluorescence emission at 513 nm (excitation wavelength = 505 nm) and compared to control sample emission.

## Claims

1. A purified polynucleotide molecule comprising a nucleic acid sequence which encodes a polypeptide having at least about 90% homology to the nucleic acid sequence SEQ ID NO:3.

2. A polynucleotide according to Claim 1 comprising a nucleic acid sequence which encodes a polypeptide selected from SEQ ID NO:3.

3. The polynucleotide of Claim 2, wherein the polynucleotide sequence comprises the sequence as depicted in SEQ ID NO:1 or SEQ ID NO:2.

4. An expression vector comprising the polynucleotide of Claim 1.

5. A host cell transformed with the expression vector of Claim 4.

6. A purified polypeptide comprising the amino acid sequence as depicted in SEQ ID NO:3 or a variant of SEQ ID NO:3 having at least about 90% homology to SEQ ID NO:3.

7. A method for producing a polypeptide according to Claim 6, said method comprising the steps of:
a) culturing a host cell according to Claim 5 under conditions suitable for the expression of said polypeptide.

8. A method of identifying compounds that modulate a biological and/or pharmacological activity of a transcription factor-selective nuclear transport receptor polypeptide, comprising:
(a) combining a candidate compound modulator with a polypeptide comprising a sequence having at least about 90% homology to SEQ ID NO:3, and
(b) measuring an effect of the candidate compound modulator on the biological and/or pharmacological activity of the polypeptide.

9. A method of identifying compounds that modulate a biological and/or pharmacological activity of a transcription factor-selective nuclear transport receptor polypeptide according to Claim 8, comprising:
(a) combining a candidate compound modulator with a host-cell which expresses said polypeptide, and
(b) measuring an effect of the candidate compound modulator on the biological and/or pharmacological activity of the polypeptide.

## Patentansprüche

1. Gereinigtes Polynukleotid-Molekül, umfassend eine Nukleinsäuresequenz, die ein Polypeptid mit wenigstens etwa 90 % Homologie zu der Nukleinsäuresequenz SEQ ID NO:3 kodiert.

2. Polynukleotid gemäß Anspruch 1, umfassend eine Nukleinsäuresequenz, die ein Polypeptid, ausgewählt aus SEQ ID NO:3 kodiert.

3. Polynukleotid gemäß Anspruch 2, bei dem die Polynukleotidsequenz die Sequenz umfasst, wie sie in SEQ ID NO:1 oder SEQ ID NO:2 dargestellt ist.

4. Expressionsvektor, umfassend das Polynukleotid von Anspruch 1.

5. Wirtszelle, transformiert mit dem Expressionsvektor von Anspruch 4.

6. Gereinigtes Polypeptid, umfassend die Aminosäuresequenz, wie sie in SEQ ID NO:3 oder einer Variante von SEQ ID NO:3 dargestellt ist, mit wenigstens etwa 90 % Homologie zu SEQ ID NO:3.

7. Verfahren zur Herstellung eines Polypeptids gemäß Anspruch 6, wobei das Verfahren die Schritte enthält:
a) Kultivieren einer Wirtszelle gemäß Anspruch 5 unter Bedingungen, die zur Expression des Polypeptids geeignet sind.

8. Verfahren zur Identifizierung von Verbindungen, die eine biologische und/oder pharmakologische Aktivität eines Transkriptionsfaktor-selektiven Nukleintransportrezeptor-Polypeptides, umfassend:
a) Kombinieren eines Kandidaten-Verbindungsmodulators mit einem Polypeptid, umfassend eine Sequenz mit wenigstens etwa 90 % Homologie zu SEQ ID NO:3, und
b) Messen eines Effekts des Kandidaten-Verbindungsmodulators an der biologischen und/oder pharmakologischen Aktivität des Polypeptids.

9. Verfahren zur Identifizierung von Verbindungen, die eine biologische und/oder pharmakologische Aktivität eines Transkriptionsfaktor-selektiven Nukleintransportrezeptor-Polypeptids gemäß Anspruch 8, umfassend:
a) Kombinieren eines Kanditaten-Verbindungsmodulators mit einer Wirtszelle, die das Polypeptid exprimiert, und
b) Messen eines Effekts des Kanditaten-Verbindungsmodulators an der biologischen und/oder pharmakologischen Aktivität des Polypeptids.

## Revendications

1. Molécule de polynucléotide purifié comprenant une séquence d'acide nucléique qui code pour un polypeptide présentant un degré d'homologie d'au moins environ 90% avec la séquence d'acide nucléique SEQ ID n°3.

2. Polynucléotide selon la revendication 1 comprenant une séquence d'acide nucléique qui code pour un polypeptide choisi à partir de la SEQ ID n° 3.

3. Polynucléotide selon la revendication 2, où la séquence polynucléotidique comprend la séquence telle que décrite dans la SEQ ID n° 1 ou la SEQ ID n° 2.

4. Vecteur d'expression comprenant le polynucléotide selon la revendication 1.

5. Cellule hôte transformée avec le vecteur d'expression selon la revendication 4.

6. Polypeptide purifié comprenant la séquence d'acides aminés telle que décrite dans la SEQ ID n° 3 ou une forme variante de la SEQ ID n° 3 présentant un degré d'homologie d'au moins environ 90% avec la SEQ ID n° 3.

7. Procédé de production d'un polypeptide selon la revendication 6, ledit procédé comprenant l'étape consistant à :
a) cultiver une cellule hôte selon la revendication 5 dans des conditions appropriées pour l'expression dudit polypeptide.

8. Procédé d'identification de composés qui modulent une activité biologique et/ou pharmacologique d'un polypeptide de récepteur de transport nucléaire sélectif vis-à-vis d'un facteur de transcription, comprenant :
a) l'association d'un composé modulateur candidat avec un polypeptide comprenant une séquence présentant un degré d'homologie d'au moins environ 90% avec la SEQ ID n° 3, et
b) la mesure d'un effet du composé modulateur candidat sur l'activité biologique et/ou pharmacologique du polypeptide.

9. Procédé d'identification de composés qui modulent une activité biologique et/ou pharmacologique d'un polypeptide de récepteur de transport nucléaire sélectif vis-à-vis d'un facteur de transcription selon la revendication 8, comprenant :
a) l'association d'un composé modulateur candidat avec une cellule hôte qui exprime ledit polypeptide, et
b) la mesure d'un effet du composé modulateur candidat sur l'activité biologique et/ou pharmacologique du polypeptide.
